# EUROPÄISCHE PATENTSCHRIFT

(11) **EP 1 642 537 B1**
(45) Veröffentlichungstag und Bekanntmachung des Hinweises auf die Patenterteilung: **09.04.2008**
(21) Anmeldenummer: 05021440.2
(22) Anmeldetag: 30.09.2005
(51) Int. Cl.: A61B 17/12, A61M 25/10, A61M 1/36

(54) **Mehrlumige Aortenkanüle**
Multi-lumen aortic cannula
Canule aortique à plusieurs lumens

(30) Priorität: 01.10.2004 DE 102004047970
(43) Veröffentlichungstag der Anmeldung: 05.04.2006
(73) Patentinhaber: Sorin Group Deutschland GmbH, 80939 München (DE)
(72) Erfinder: Hahn, Andreas, Dr., 82335 Berg (DE); Göllner, Marcus, 80797 München (DE); Wetzig, Michael, 80339 München (DE)
(74) Vertreter: HOFFMANN EITLE

(56) Entgegenhaltungen:
- US-A- 5 425 708
- US-A- 5 868 703
- US-A1- 2001 023 357
- US-A1- 2002 111 583
- US-A1- 2002 151 869
- US-A1- 2003 167 038
- US-B1- 6 228 053

## Beschreibung

Die vorliegende Erfindung betrifft eine mehrlumige Aortenkanüle mit einem Okklusionsballon für die Okklusion der Aorta ascendens und die Zuführung von einerseits Blut in den Aortenbogen und andererseits Kardioplegielösung in die Aorta ascendens.

Im Stand der Technik sind verschiedene Aortenkanülen für den Verschluss der Aorta ascendens und für die Zuführung von Kardioplegielösung einerseits und Blut andererseits bekannt. Der Verschluss erfolgt bei den hier in Rede stehenden Kanülen mit Hilfe eines Ballons, dem ein Dilatationsmedium zugeführt wird. Gegenüber der vor der Entwicklung von Okklusionsballons üblichen Gefäßquerklemmung zeichnet sich die intraluminale Okklusion mittels Ballon dadurch aus, dass sie besonders schonend ist.

So ist aus EP 0 892 652 A eine mehrlumige Aortenkanüle mit Okklusionsballon bekannt, bei der in der Manteloberfläche eines schlauchartigen Kanülengrundkörpers vor dem Okklusionsballon Austrittsöffnungen für das durch das Perfusionslumen zugeführte Blut vorgesehen sind. Ein Plegielumen für die Zuführung von Kardioplegielösung durchdringt den Ballon und besitzt eine in Bezug auf die Strömungsrichtung hinter dem Okklusionsballon liegende Austrittsöffnung. Für die Dilatation des Okklusionsballons ist ein Lumen für die Zuführung einer Dilatationslösung integriert, das sich in das Innere des Okklusionsballons hinein öffnet.

US 5.425.708 beschreibt eine Ballonokklusionskanüle, bei der das Lumen, durch das der Aorta Blut zugeführt wird, den Okklusionsballon durchdringt, während das für Plegielösung vorgesehene Lumen sich in Strömungsrichtung des Blutes gesehen vor dem Ballon zur Aorta ascendens öffnet.

Auch EP 1 086 717 A beschreibt eine aortale Ballonokklusionskanüle. Diese Kanüle besitzt zwei Okklusionsballons auf einem Kanülengrundkörper, in dem neben dem Perfusionslumen für die Zuführung des Blutes ein Lumen für die Zuführung von Kardioplegielösung und jeweils ein Lumen für jeden der Ballons zur Zuführung einer Dilatationsflüssigkeit vorgesehen ist. Sowohl das Plegielumen als auch das Perfusionslumen durchdringen die beiden Ballons, wobei aber nur das Perfusionslumen eine Öffnung sowohl in Strömungsrichtung des Blutes hinter den Okklusionsballons als auch vor den Okklusionsballons besitzt. Das Plegielumen besitzt eine Öffnung nur in Strömungsrichtung hinter den beiden Okklusionsballons. In einem beschriebenen Anwendungsfall wird der durch die Okklusionsballons reichende Teil des Perfusionslumens mit Hilfe eines Schiebers verschlossen, die Aorta mit Hilfe der beiden Ballons okkludiert, dem Herzen über das Plegielumen Kardioplegielösung und über das Perfusionslumen Blut zugeführt. Im Vordergrund steht bei dieser bekannten aortalen Ballonokklusionskanüle jedoch, dass zwischen den beiden Ballons ein begrenzter Bereich der Aorta festgelegt wird, der auch dann blutfrei gehalten werden kann, wenn der Schieber geöffnet wird und Blut durch den die beiden Ballons durchdringenden Abschnitt des Perfusionslumens strömt. Der abgetrennte Bereich steht dann für das Annähen von Venen-Bypässen zur Verfügung, während zur Verkürzung der Ischämiezeit die Aortenwurzelperfusion über den die Ballons durchdringenden Teil des Perfusionslumens vollzogen werden kann.

Während bei den zuvor beschriebenen Aortenkanülen Okklusionsballons vorgesehen sind und damit zusammenhängende Aspekte im Vordergrund stehen, beschreibt WO 01/34239 A eine reine Perfusionskanüle für die Zuführung von Blut in die Aorta ohne Okklusionsballon. Das Augenmerk liegt bei dieser bekannten Aortenkanüle auf der Vermeidung eines zu starken und gerichteten Blutstroms, der in Anbetracht der hohen Fördermengen aus der Austrittsöffnung einer Aortenkanüle austritt. Zur Vermeidung einer die Aorta schädigenden Einwirkung des Blutstroms ist bei der Aortenkanüle gemäß WO 01/34239 A eine löffelförmige Lippe vorgesehen, die vor der Austrittsöffnung angeordnet ist und den im wesentlichen senkrecht zur Aorta zugeführten Blutstrom nach dem Austreten durch die Austrittsöffnung in die gewünschte Strömungsrichtung ablenkt, dabei aber eine U-förmige Verteilung bewirkt, so dass ein Auftreffen auf die Aortenwand und eine Beschädigung derselben vermieden wird.

Eine weitere Kanüle ist aus US 2004/0162519 bekannt, bei der drei Lumen vorgesehen sind und ein Ballon auf dem das Blut führenden Lumen angeordnet ist. Ein Lumen für Kardioplegielösung endet vor dem Ballon und ein weiteres Lumen dient zur Zuführung von Dilatationsflüssigkeit für die Dilatation des Ballons. Die Anordnung des Ballons auf dem blutführenden Lumen ist in Anbetracht der für die Zuführung von erforderlichen Dimensionierung ungünstig und verhindert eine optimierte Gestaltung des Ballons und der gesamten Kanülenspitze.

Denn neben den zuvor angesprochenen Aspekten, die bei Aortenkanülen eine Rolle spielen, ist grundsätzlich zu beachten, dass es für eine gute Handhabbarkeit einer Aortenkanüle erforderlich ist, dass die Gestaltung an ihrem distalen Ende das Einführen der Kanüle in die Aorta bzw. das Entfernen nicht übermäßig behindern darf. Dennoch muss eine zuverlässige Okklusion der Aorta und eine sichere Positionierung der Kanülenspitze in der Aorta erreicht werden. Daneben muss die Zuführung des Blutes so vonstatten gehen, dass weder das zugeführte Blut, noch die Innenwand der Aorta geschädigt werden.

Vor diesem Hintergrund besteht das der Erfindung zugrundeliegende Ziel darin, eine mehrlumige Aortenkanüle mit Okklusionsballon anzugeben, deren Gestaltung im Hinblick auf die Handhabung durch den Arzt, den sicheren Sitz in der okkludierten Aorta und die schonende Zuführung des Blutes in abgewogener Abstimmung vorteilhaft ist.

Dieses Ziel wird erfindungsgemäß erreicht durch eine mehrlumige Aortenkanüle mit einem Okklusionsballon für den Verschluss der Aorta ascendens, und mit einem Kanülengrundkörper, an dessen distalen Ende der Okklusionsballon angeordnet ist und der zumindest die folgenden Lumen aufweist: ein Perfusionslumen für die Zuführung von Blut in die Aorta, das in Strömungsrichtung des zugeführten Blutes vor dem Okklusionsballon mindestens eine Austrittsöffnung für das zugeführte Blut aufweist, das aber den Okklusionsballon nicht durchdringt, ein zweites Lumen für die Zuführung von Kardioplegielösung in die Aorta ascendens, das sich durch den Okklusionsballon erstreckt und das in Strömungsrichtung der Plegielösung hinter dem Okklusionsballon mindestens eine Austrittsöffnung für die zugeführte Plegielösung aufweist, und ein drittes Lumen für die Zuführung eines Mediums zur Dilatation des Okklusionsballons, das mindestens eine sich in das Innere des Okklusionsballons öffnende Austrittsöffnung für das zugeführte Dilatationsmedium aufweist. Gemäß der Erfindung liegt der Rand der Austrittsöffnung des Perfusionslumens im wesentlichen in einer Ebene (Öffnungsebene), ist ein zur Austrittsöffnung auslaufender konkaver Wandbereich im Inneren des Perfusionslumens zur Ablenkung des durch das Perfusionslumen zugeführten Blutes in Richtung der Austrittsöffnung vorgesehen und ist die Austrittsöffnung des Perfusionslumens in Bezug auf das zweite Lumen an dem Kanülengrundkörper derart angeordnet, dass die Öffnungsebene der Austrittsöffnung des Perfusionslumens mit der Längsachse des den Okklusionsballon durchdringenden Abschnitts des zweiten Lumens einen Winkel von etwa 60° bis etwa 120°, vorzugsweise 90° ± 10° einnimmt. Ferner ist erfindungsgemäß der Okklusionsballon in den für die Berührung mit der Aorteninnenwand vorgesehenen Bereichen im Querschnitt abgeflacht.

Die erfindungsgemäße Lösung stellt eine vorteilhafte Merkmalszusammenstellung dar, mit der die oben angegebenen Ziele erreicht werden. Denn aufgrund der durch die Abflachung erzeugten großen Außenoberflächen des Okklusionsballons wird eine in besonderem Maße wirksame Auflagefläche an der Aorteninnenwand geschaffen, so dass der Teil des Plegielumens, an dem der Okklusionsballon befestigt ist, sehr kurz gehalten werden kann, ohne dass ein sicherer Halt der Kanüle in der okkludierten Aorta beeinträchtigt wird. Die Verkürzung gerade dieses Teils der Kanülenspitze trägt zur einfachen Handhabung bei der Einführung der Aortenkanüle in die Aorta bei. Eine für die Halterung optimierte Gestaltung des Ballons ist deshalb möglich, weil der Ballon erfindungsgemäß auf dem Plegielumen angeordnet ist und dadurch ein größerer Gestaltungsspielraum gegebenen ist, da das Plegielumen aufgrund seiner geringeren Größe vorteilhaft ist. Daneben endet erfindungsgemäß das Perfusionslumen unmittelbar vor dem Okklusionsballon und besitzt dort eine Austrittsöffnung, die an dem Kanülengrundkörper angeordnet ist und deren Rand im wesentlichen in einer Ebene liegt. Deshalb stellt auch dieser Bereich der Kanülenspitze mangels vorspringender Teile bei der Einführung der Aortenkanüle in die Aorta keine Behinderung dar. Außerdem bietet diese Gestaltung der Austrittsöffnung einen hohen Schutz gegen Beschädigung und damit verbundene Ablösung (Abbrechen, Absplittern) von Teilen, die ein kritisches Sicherheitsrisiko darstellen. Schließlich besitzt, um eine schonende Zuführung des Blutes zu gewährleisten, die erfindungsgemäße Aortenkanüle am Ende des Perfusionslumens im Inneren einen konkaven Bereich, der sich zur Blutaustrittsöffnung hin öffnet und das Blut in diese Richtung schonend ablenkt. Damit ist verbunden, dass die Austrittsrichtung der zugeführten Blutes im wesentlichen der üblichen Strömungsrichtung des Blutes in der Aorta entspricht, so dass eine Beschädigung der Aortenwand oder die mit Kalkablösungen verbundenen Gefahren weitestgehend vermieden wird.

Weitere vorteilhafte Ausgestaltungen der erfindungsgemäßen Kanüle ergeben sich aus den Unteransprüchen und der folgenden Beschreibung von Ausführungsbeispielen der Erfindung, bei der Bezug genommen wird auf
- Figur 1,: die einen Querschnitt des distalen Endes einer erfindungsgemäßen Aortenkanüle mit Okklusionsballon und Kanülengrundkörper zeigt,
- Figur 2,: die einen Querschnitt durch den Kanülengrundkörper senkrecht zu dessen Längsachse zeigt, und
- Figur 3,: die einen Querschnitt einer weiteren Ausgestaltung des Okklusionsballons zeigt.

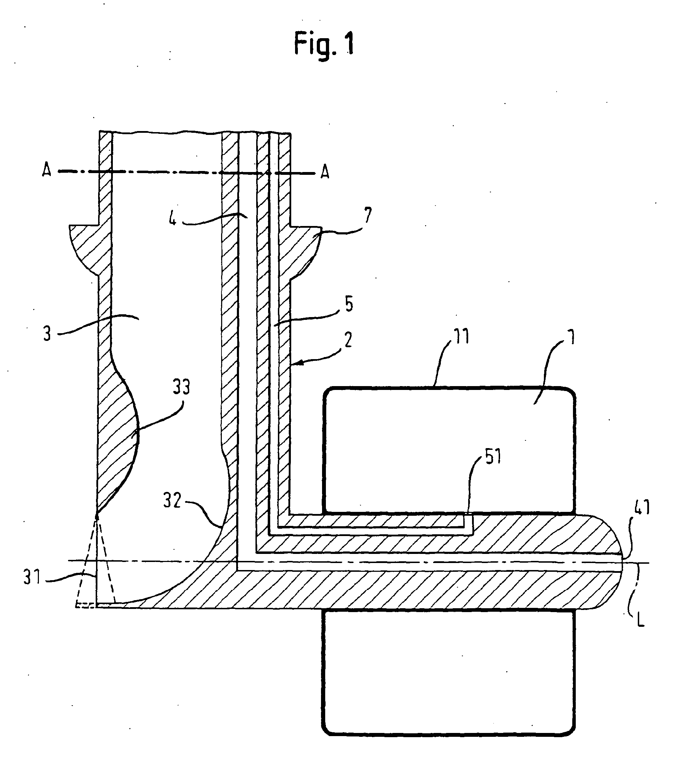

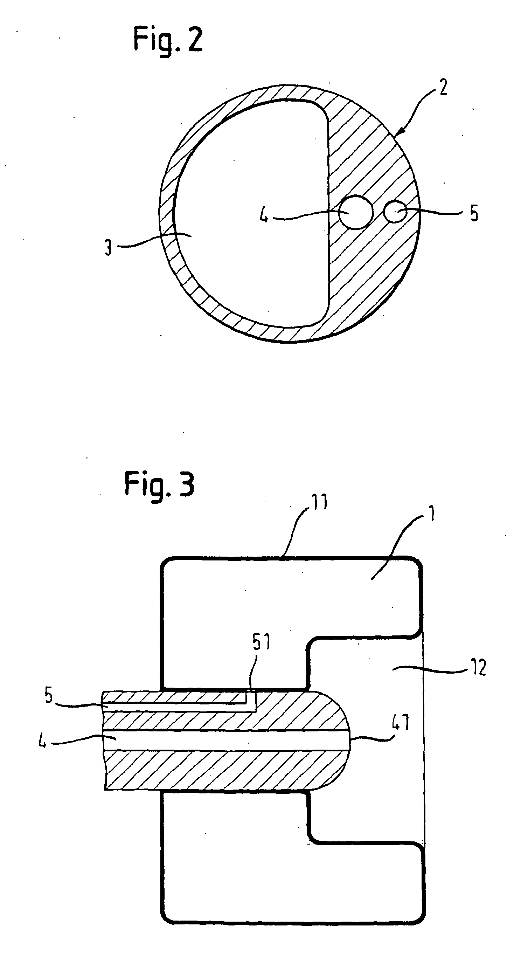

Figur 1 zeigt das distale Ende einer erfindungsgemäßen Aortenkanüle mit einem Okklusionsballon 1 und einem Kanülengrundkörper 2, der mehrere Lumen in integrierter Form aufweist. Das Perfusionslumen 3 dient zur Zuführung von Blut in die Aorta. Das zweite Lumen 4 dient zur Zuführung von Kardioplegielösung in die Aorta ascendens. Das dritte Lumen 5 dient zur Zuführung von Dilatationsflüssigkeit in den Okklusionsballon 1. Figur 2 zeigt einen Querschnitt durch den Kanülengrundkörper 2 an der in Figur 1 mit A-A gekennzeichneten Stelle. Die Größe, die Form und die Anordnung der Lumen im Einzelnen ist in Figur 2 nur beispielhaft und schematisch gezeigt. Grundsätzlich ist aber bei einer erfindungsgemäßen Aortenkanüle der Querschnitt des Perfusionslumens 3 (erstes Lumen) sehr viel größer als die Querschnitte des zweiten Lumens 4 (Plegielumen) und des dritten Lumens 5 (Dilatationslumen).

Der Querschnitt, der in Figur 2 gezeigt ist, macht deutlich, dass in dem Kanülengrundkörper 2 des Ausführungsbeispiels einer erfindungsgemäßen Aortenkanüle ausreichend Platz zur Verfügung steht für weitere Lumen, beispielsweise für die Zuführung oder Entnahme von Flüssigkeiten oder zur Druckmessung am distalen Ende der Kanüle. Welche zusätzlichen Lumen vorgesehen werden, richtet sich nach dem konkret in Betracht gezogenen Anwendungsfall; erfindungsgemäß sind aber die oben erwähnten drei Lumen, also das Perfusionslumen, das Plegielumen und das Dilatationslumen immer vorhanden und in den Kanülengrundkörper 2 integriert, wie die Figuren 1 und 2 zum Ausdruck bringen.

Wie Figur 1 zeigt, besitzt das Perfusionslumen 3 für die Zuführung von Blut in die Aorta eine Austrittsöffnung 31, deren Rand im wesentlichen in einer Ebene liegt. Diese Ebene ist, wie Figur 1 zeigt, am Kanülengrundkörper 2 angeordnet, so dass die Außenwand des Kanülenrundkörpers 2 zumindest bereichsweise, beispielweise entlang einer Linie, wie etwa Figur 1 zeigt, mit der Austrittsöffnungsebene zusammenfällt. Bei dieser Ausgestaltung wird auf einen aus dem Bereich des Kanülengrundkörpers rohrartig vorspringender Abschnitt des ersten Lumens 3 verzichtet.

Im Inneren des Perfusionslumens 3 ist ein konkaver Wandbereich 32 ausgebildet, der zur Austrittsöffnung 31 hin ausläuft und so gestaltet ist, dass er das durch das Perfusionslumen 3 zugeführte Blut in Richtung auf die Austrittsöffnung 31 ablenkt. Durch den zur Austrittsöffnung auslaufenden konkaven Wandbereich 32 des Perfusionslumens 3 wird die Strömungsrichtung des Blutes, die in der Darstellung der Figur 1 zunächst von oben nach unten verläuft, so geändert, dass das Blut im wesentlichen senkrecht zur Öffnungsebene der Austrittsöffnung 31 strömt, wenn es aus dem Perfusionslumen 3 in die Aorta austritt, was in Figur 1 einer Strömung nach links entspricht.

Die Öffnungsebene der Austrittsöffnung 31 des Perfusionslumens 3 steht bei dem in Figur 1 gezeigten Ausführungsbeispiel der Erfindung senkrecht zur Längsachse L eines Abschnitts des zweiten Lumens 4, der den Okklusionsballon 1 durchdringt. Auf der Seite des Okklusionsballons 1, die in Strömungsrichtung der Plegielösung hinter dem Ballon und damit in Figur 1 rechts davon liegt, besitzt der den Okklusionsballon 1 durchdringende Abschnitt des zweiten Lumens 4 eine Austrittsöffnung 41, aus der die zugeführte Kardioplegielösung in die Aorta ascendens austritt.

Wie in Figur 1 gestrichelt angedeutet, kann die Öffnungsebene der Austrittsöffnung 31 in Abwandlung des gezeigten Ausführungsbeispiels einen Winkel von 60° bis 120°, vorzugsweise 90° ± 10° mit der Längsachse L des den Okklusionsballon durchdringenden Abschnitts des zweiten Lumens 4 einnehmen. In jedem Fall aber liegt der Rand der Austrittsöffnung 31 des Perfusionslumens 3 im Wesentlichen in einer Ebene (Öffnungsebene).

An der Wand des den Okklusionsballon 1 durchdringenden Abschnitts des zweiten Lumens 4 ist der Okklusionsballon 1, wie Figur 1 zeigt, derart angeordnet, dass das dritte Lumen 5 mit dem Inneren des Ballons 1 verbunden ist. Dazu weist das dritte Lumen 5 mindestens eine Austrittsöffnung 51 auf, aus der ein zugeführtes Dilatationsmedium in das Innere des Okklusionsballons 1 eindringt und diesen dilatiert. In Figur 1 ist der dilatierte Zustand des Okklusionsballons 1 dargestellt. Aus der Abbildung der Figur 1 ergibt sich, dass bei dem hier beschriebenen Ausführungsbeispiel der Erfindung der Okklusionsballon 1 im Querschnitt abgeflachte Bereiche 11 dort aufweist, wo er mit der Aorteninnenwand (nicht dargestellt) in Berührung gerät.

Da Figur 1 einem Querschnitt durch das distale Ende eines Ausführungsbeispiels der Erfindung zeigt, sei an dieser Stelle angemerkt, dass sich der Okklusionsballon 1 um die Wandung des Abschnitts des zweiten Lumens 4 erstreckt, der den Okklusionsballon 1 durchdringt. Damit besitzt der erfindungsgemäße Okklusionsballon 1 eine hohlzylindrische Grundform, bei der die äußere Zylinderwand die im Querschnitt abgeflachten Bereiche 11 für die Berührung mit der Aorteninnenwand bildet, während die innere Zylinderwand des Hohlzylinders der Anordnung und Befestigung an der Außenwand des das zweite Lumen 4 umschließenden Kanülenkörpers dient, der bei dem hier beschriebenen Ausführungsbeispiel ebenfalls eine zylindrische Grundform aufweist, wie Figur 1 zeigt.

Die Fixierung einer erfindungsgemäßen Kanüle kann bekanntermaßen durch eine Naht an einem Wulstkragen 7 erfolgen; jedoch wird diese Fixierung unterstützt oder, bei geeigneter Auslegung, ersetzt durch die fixierende Wirkung des Ballons selbst. Da die ergänzende bzw. alleinige Fixierung der erfindungsgemäßen Aortenkanüle durch im Querschnitt abgeflachten Bereiche 11 des Okklusionsballons 1 erreicht wird, kann die Gesamtlänge des den Ballon tragenden Teils der Kanüle sehr kurz ausgelegt werden. Deshalb ist die Gefahr einer Beschädigung der Herzklappe, auf die dieser Teil der Kanüle im eingeführten Zustand gerichtet ist, sehr gering, da dieser kurze Teil der erfindungsgemäßen Kanüle die Herzklappe beim Einführen der Kanüle in die Aorta nicht erreicht. Durch die kurze Gestaltung wird der Operationsbereich nicht beeinträchtigt und die Handhabung vereinfacht. Außerdem ist zu beachten, dass durch die erfindungsgemäße Gestaltung der Kanüle zwei separate Kanülen und eine Gefäßklemme ersetzt werden. Schließlich kann bei einer erfindungsgemäßen Kanüle ein weiteres Lumen zur Druckmessung vorgesehenen werden.

Die Gefahr einer Berührung mit der Herzklappe kann weiter herabgesetzt werden, wenn der Okklusionsballon 1 mit einem Querschnitt ausgestattet wird, der in Figur 3 gezeigt ist. In der Darstellung der Figur 3 ist erkennbar, dass bei diesem Ausführungsbeispiel der Erfindung der den Okklusionsballon durchdringende Abschnitt des zweiten Lumens 4 sich nur etwas über die Mitte der Länge des den Okklusionsballon bildenden Hohlzylinders erstreckt. Der Okklusionsballon 1 umfasst aber einen zurückspringenden Bereich 12, der gewährleistet, dass das zweite Lumen 4 den Okklusionsballon 1 tatsächlich durchdringt, d.h. die Austrittsöffnung 41 des zweiten Lumens 4 nicht abgedeckt wird und die Plegielösung aus dem zweiten Lumen 4 austreten kann. Trotz der Verkürzung im Inneren ist der erfindungsgemäße großflächige Auflagebereich 11 am äußeren Umfang des Okklusionsballons 1 gegeben, wodurch der sichere Sitz der Kanüle in der Aorta gewährleistet wird. Die Verkürzung des den Okklusionsballon durchdringenden Teils des zweiten Lumens 4 wirkt sich auch positiv auf die Handhabung der erfindungsgemäßen Kanüle aus, da dieser Teil sich umso einfacher in die Aorta einführen lässt, je kürzer er ist.

Wie aus Figur 1 zu entnehmen ist, umfassen die beschriebenen Ausführungsbeispiele der Erfindung einen Wandvorsprung 33 im Inneren des Perfusionslumens 3. Dieser Wandvorsprung 33 ist so ausgebildet und dimensioniert, dass das zugeführte Blut in Richtung auf den konkaven Wandbereich 32, der zur Austrittsöffnung 31 des Perfusionslumens 3 hin ausläuft, umgelenkt wird. Dazu ist der Wandvorsprung 33 im wesentlichen auf der dem konkaven Wandbereich 32 gegenüber liegenden Innenwand des Perfusionslumens 3 aber in Strömungsrichtung des zugeführten Blutes vor dem konkaven Wandbereich 32 angeordnet. Der Wandvorsprung 33 ist vorzugsweise abgerundet, so dass bei der Umlenkung keine schädigende Wirkung auf das zugeführte Blut ausgeübt wird. Der Wandvorsprung 33 ist vorteilhafterweise so groß, dass er im Bereich seiner maximalen Ausdehnung in das Innere des Perfusionslumens 3 hinein den Durchtrittsquerschnitt des Perfusionslumens 3 um bis zu 30 % reduziert, insbesondere im Vergleich zum Durchtrittsquerschnitt der Austrittsöffnung 31 des Perfusionslumens 3. Eine weitergehende Reduzierung des Durchtrittsquerschnitts im Bereich des Wandvorsprungs 33 ist in Abhängigkeit von den konstruktiven Einzelheiten der Aortenkanüle ggf. zweckdienlich.

Das in Figur 1 gezeigte distale Ende eines Ausführungsbeispiels der Erfindung kann auch als Kanülenkopf ausgebildet sein, der am Ende eines schlauchartigen Abschnitts der Aortenkanüle angebracht ist. Das bedeutet, dass etwa im Bereich der Linie A-A ein geeigneter Anschluss für einen flexiblen schlauchartigen Abschnitt der erfindungsgemäßen Aortenkanüle vorzusehen ist, auf den der schlauchartige Abschnitt aufgesteckt wird. Sowohl der Kanülenkopf als auch der schlauchartige Abschnitt der erfindungsgemäßen Aortenkanüle weisen zumindest die drei eingangs erwähnten Lumen, nämlich das Perfusionslumen, das Plegielumen und das Dilatationslumen auf. Dabei ist die Geometrie, insbesondere der Querschnitt des schlauchartige Abschnitts der Kanüle an den Anschlussabschnitt im Bereich A-A des Kanülenkopfes angepasst, besteht aber vorteilhaft aus einem anderen, z.B. einem flexiblen Material. Der Kanülenkopf ist vorzugsweise aus einem im Vergleich dazu starreren Material ausgebildet, was das Einführen der erfindungsgemäßen Kanüle in die Aorta erleichtert. Durch die zuvor beschriebene Trennung in Kanülenkopf und schlauchartigen Abschnitt ist die Verwendung unterschiedlicher Materialien möglich.

## Patentansprüche

1. Mehrlumige Aortenkanüle mit
einem Okklusionsballon (1) für den Verschluss der Aorta ascendens, und
einem Kanülengrundkörper (2), an dessen distalen Ende der Okklusionsballon (1) angeordnet ist und der zumindest aufweist:
- ein Perfusionslumen (3) für die Zuführung von Blut in die Aorta, das in Strömungsrichtung des zugeführten Blutes vor dem Okklusionsballon (1) mindestens eine Austrittsöffnung (31) für das zugeführte Blut aufweist und das den Okklusionsballon (1) nicht durchdringt,
- ein zweites Lumen (4) für die Zuführung von Kardioplegielösung in die Aorta ascendens, das sich durch den Okklusionsballon (1) erstreckt und das in Strömungsrichtung der Plegielösung hinter dem Okklusionsballon (1) mindestens eine Austrittsöffnung (41) für die zugeführte Plegielösung aufweist, und
- ein drittes Lumen (5) für die Zuführung eines Mediums zur Dilatation des Okklusionsballons (1), das mindestens eine sich in das Innere des Okklusionsballons öffnende Austrittsöffnung (51) für das zugeführte Dilatationsmedium aufweist,
**dadurch gekennzeichnet, dass**
- der Rand der Austrittsöffnung (31) des Perfusionslumens im Wesentlichen in einer Ebene (Öffnungsebene) liegt,
- ein zur Austrittsöffnung auslaufender konkaver Wandbereich (32) im Inneren des Perfusionslumens (3) zur Ablenkung des durch das Perfusionslumen zugeführten Blutes in Richtung der Austrittsöffnung (31) vorgesehen ist, wobei die Strömungsrichtung des Blutes so geändert wird, dass das Blut im wesentlichen senkrecht zur Öffnungsebene der Austrittsöffnung (31) strömt,
- die Austrittsöffnung (31) des Perfusionslumens (3) in Bezug auf das zweite Lumen (4) an dem Kanülengrundkörper (2) derart angeordnet ist, dass die Öffnungsebene der Austrittsöffnung (31) des Perfusionslumens (3) mit der Längsachse (L) des den Okklusionsballon durchdringenden Abschnitts des zweiten Lumens einen Winkel von 60° bis 120°, vorzugsweise 90°± 10° einnimmt, und
- der Okklusionsballon (1) in den für die Berührung mit der Aorteninnenwand vorgesehenen Bereichen (11) zumindest in einem Querschnitt abgeflacht ist.

2. Mehrlumige Aortenkanüle nach Anspruch 1, **dadurch gekennzeichnet, dass** der Okklusionsballon (1) eine hohlzylindrische Grundform aufweist und die äußere Mantelfläche des hohlzylindrischen Okklusionsballons (1) den für die Berührung mit der Aorteninnenwand vorgesehenen Bereich (11) bildet.

3. Mehrlumige Aortenkanüle nach Anspruch 2, **dadurch gekennzeichnet, dass** sich das zweite Lumen (4) entlang der Längsachse des hohlzylindrischen Okklusionsballons (1) erstreckt.

4. Mehrlumige Aortenkanüle nach Anspruch 2 oder 3, **dadurch gekennzeichnet, dass** der hohlzylindrische Okklusionsballon einen zurückspringenden Bereich (12) aufweist, so dass der den Okklusionsballon durchdringende Abschnitt des zweiten Lumens (4) kürzer ist als die äußere Mantelfläche (11) des hohlzylindrischen Okklusionsballons (1), die für die Berührung mit der Aorteninnenwand vorgesehen ist.

5. Mehrlumige Aortenkanüle nach einem der vorangegangenen Ansprüche, **dadurch gekennzeichnet, dass** im Inneren des Perfusionslumens (3) ein Wandvorsprung (33) für die Umlenkung des durch das erste Lumen zugeführte Blut in Richtung auf den konkaven Wandbereich (32) vorgesehen ist.

6. Mehrlumige Aortenkanüle nach Anspruch 5, **dadurch gekennzeichnet, dass** der Wandvorsprung (33) im Inneren des Perfusionslumens (3) den Durchtrittsquerschnitt des Perfusionslumens (3) um bis zu 30%, insbesondere im Vergleich zum Durchtrittsquerschnitt der Austrittsöffnung (31) des Perfusionslumens (3) verkleinert.

7. Mehrlumige Aortenkanüle nach Anspruch 5 oder 6, **dadurch gekennzeichnet, dass** der Wandvorsprung (33) an einer Stelle der Innenwand des Perfusionslumens (3) angeordnet ist, der dem konkaven Wandbereich (32) der Innenwand des Perfusionslumens (3) gegenüber aber in Strömungsrichtung des zugeführten Blutes vor dem konkaven Wandbereich (32) liegt.

8. Mehrlumige Aortenkanüle nach einem der Ansprüche 5, 6 oder 7, **dadurch gekennzeichnet, dass** der Wandvorsprung (33) abgerundet ist.

9. Mehrlumige Aortenkanüle nach einem der vorangegangenen Ansprüche, **dadurch gekennzeichnet, dass** weitere Lumen, beispielsweise für Druckmessungen vorgesehen sind.

## Claims

1. Multi-lumen aortic cannula having an occlusion balloon (1) for closure of the aorta ascendens, and a cannula base body (2), on the distal end of which is arranged the occlusion balloon (1) and which has at least:
- one perfusion lumen (3) for supplying blood to the aorta, which has at least one outlet opening (31) for the supplied blood upstream of the occlusion balloon (1) in flow direction of the supplied blood and which does not penetrate the occlusion balloon (1),
- one second lumen (4) for supplying cardioplegia solution to the aorta ascendens, which extends through the occlusion balloon (1) and which has at least one outlet opening (41) for the supplied plegia solution downstream of the occlusion balloon (1) in flow direction of the plegia solution, and
- one third lumen (5) for supplying a medium for dilation of the occlusion balloon (1), which has at least one outlet opening (51), which opens into the interior of the occlusion balloon, for the supplied dilation medium,
**characterised in that**
- the edge of the outlet opening (31) of the perfusion lumen lies essentially in one plane (opening plane),
- a concave wall region (32) discharging towards the outlet opening is provided in the interior of the perfusion lumen (3) for deflecting the blood supplied through the perfusion lumen in the direction of the outlet opening (31), wherein the flow direction of the blood is changed so that the blood flows essentially vertically to the opening plane of the outlet opening (31),
- the outlet opening (31) of the perfusion lumen (3) is arranged on the cannula base body (2) with respect to the second lumen (4) such that the opening plane of the outlet opening (31) of the perfusion lumen (3) occupies an angle of 60° to 120°, preferably 90° ± 10° with the longitudinal axis (L) of the section of the second lumen penetrating the occlusion balloon, and
- the occlusion balloon (1) is flattened in the regions (11) intended for contact with the inner wall of the aorta at least in one cross-section.

2. Multi-lumen aortic cannula according to claim 1, **characterised in that** the occlusion balloon (1) has a hollow cylindrical basic shape and the outer shell surface of the hollow cylindrical occlusion balloon (1) forms the region (11) intended for contact with the inner wall of the aorta.

3. Multi-lumen aortic cannula according to claim 2, **characterised in that** the second lumen (4) extends along the longitudinal axis of the hollow cylindrical occlusion balloon (1).

4. Multi-lumen aortic cannula according to claim 2 or 3, **characterised in that** the hollow cylindrical occlusion balloon has a rebounding region (12), so that the section of the second lumen (4) penetrating the occlusion balloon is shorter than the outer shell surface (11) of the hollow cylindrical occlusion balloon (1), which is intended for contact with the inner wall of the aorta.

5. Multi-lumen aortic cannula according to one of the preceding claims, **characterised in that** a wall projection (33) for the deflection of the blood supplied through the first lumen in the direction of the concave wall region (32) is provided in the interior of the perfusion lumen (3).

6. Multi-lumen aortic cannula according to claim 5, **characterised in that** the wall projection (33) in the interior of the perfusion lumen (3) reduces the passage cross-section of the perfusion lumen (3) by up to 30%, in particular compared to the passage cross-section of the outlet opening (31) of the perfusion lumen (3).

7. Multi-lumen aortic cannula according to claim 5 or 6, **characterised in that** the wall projection (33) is arranged at one point of the inner wall of the perfusion lumen (3), which lies opposite the concave wall region (32) of the inner wall of the perfusion lumen (3), but upstream of the concave wall region (32) in flow direction of the supplied blood.

8. Multi-lumen aortic cannula according to one of claims 5, 6 or 7, **characterised in that** the wall projection (33) is rounded.

9. Multi-lumen aortic cannula according to one of the preceding claims, **characterised in that** further lumens are provided, for example for pressure measurements.

## Revendications

1. Canule aortique à plusieurs lumens, comprenant
un ballon d'occlusion (1) pour l'obturation de l'aorta ascendens, et
un corps de base de canule (2), à l'extrémité distale duquel est disposé le ballon d'occlusion (1) et présentant au moins :
- un lumen de perfusion (3) pour l'amenée de sang dans l'aorte, présentant, en observant dans la direction d'écoulement du sang amené, en amont du ballon d'occlusion (1) au moins une ouverture de sortie (31) pour le sang amené et ne traversant pas le ballon d'occlusion (1),
- un deuxième lumen (4) pour l'amenée de solutions de cardioplégie dans l'aorta ascendens, s'étendant à travers le ballon d'occlusion (1) et présentant, en observant dans la direction d'écoulement de la solution plégique, en aval du ballon d'occlusion (1), au moins une ouverture de sortie (41) pour la solution plégique amenée, et
- un troisième lumen (5) pour l'amenée d'un fluide pour la dilation du ballon d'occlusion (1), présentant au moins une ouverture de sortie (51), ouvrant vers l'intérieur du ballon d'occlusion (1), pour le fluide de dilatation amené,
**caractérisée en ce que**
- le bord de l'ouverture de sortie (31) du lumen de perfusion est situé sensiblement dans un plan (plan d'ouverture),
- une zone de paroi (32) concave, évoluant vers l'ouverture de sortie, est prévue à l'intérieur de lumen de perfusion (3), pour dévier le sang, amené par le lumen de perfusion, dans la direction de l'ouverture de sortie (31), la direction d'écoulement du sang étant modifiée de manière que le sang s'écoule sensiblement perpendiculairement au plan d'ouverture de l'ouverture de sortie (31),
- l'ouverture de sortie (31) du lumen de perfusion (3) est disposée, par rapport au deuxième lumen (4) sur le corps de base de canule (2), de manière que le plan d'ouverture de l'ouverture de sortie (31) du lumen de perfusion (3) fasse, avec l'axe longitudinal (L) du tronçon, traversant le ballon d'occlusion, du deuxième lumen, un angle compris dans la fourchette allant de 60° à 120°, de préférence un angle de 90° ± 10°, et
- le ballon d'occlusion (1), dans les zones (11), prévues pour le contact avec la paroi intérieure d'aorte, est aplati au moins en une section transversale.

2. Canule aortique à plusieurs lumens selon la revendication 1, **caractérisée en ce que** le ballon d'occlusion (1) présente une forme de base cylindrique creuse et la surface d'enveloppe extérieure du ballon d'occlusion (1) cylindrique creux forme la zone (11) prévue pour le contact avec la paroi intérieure d'aorte.

3. Canule aortique à plusieurs lumens selon la revendication 2, **caractérisée en ce que** le deuxième lumen (4) s'étend le long de l'axe longitudinal du ballon d'occlusion (1) cylindrique creux.

4. Canule aortique à plusieurs lumens selon la revendication 2 ou 3, **caractérisée en ce que** le ballon d'occlusion creux présente une zone (12) en retrait, de manière que le tronçon, pénétrant le ballon d'occlusion, du deuxième lumen (4) soit plus court que la surface d'enveloppe extérieure (11) du ballon d'occlusion (1) cylindrique creux, prévue pour le contact avec la paroi intérieure d'aorte.

5. Canule aortique à plusieurs lumens selon l'une des revendications précédentes, **caractérisée en ce qu'**à l'intérieur du lumen de perfusion (3) est prévue une saillie de paroi (33) pour la déviation du sang amené à travers le premier lumen, afin de l'amener dans la direction de la zone de paroi (32) concave.

6. Canule aortique à plusieurs lumens selon la revendication 5, **caractérisée en ce que** la saillie de paroi (33) à l'intérieur du lumen de perfusion (3) diminue la section transversale de passage du lumen de perfusion (3) d'une valeur allant jusqu'à 30 %, en particulier par rapport à la section transversale de passage de l'ouverture de sortie (31) du lumen de perfusion (3).

7. Canule aortique à plusieurs lumens selon la revendication 5 ou 6, **caractérisée en ce que** la saillie de paroi (33) est disposée en un emplacement de la paroi intérieure du lumen de perfusion (3) qui est situé en regard de la zone de paroi (32) concave de la paroi intérieure du lumen de perfusion (3) mais en amont de la zone de paroi (32) concave en observant dans la direction d'écoulement du sang amené.

8. Canule aortique à plusieurs lumens selon l'une des revendications 5, 6 ou 7, **caractérisée en ce que** la saillie de paroi (33) est arrondie.

9. Canule aortique à plusieurs lumens selon l'une des revendications précédentes, **caractérisée en ce que** d'autres lumens sont prévus, par exemple pour procéder à des mesures de pression.
